# EUROPÄISCHE PATENTANMELDUNG

(11) **EP 0 823 414 A1**
(43) Veröffentlichungstag der Anmeldung: **11.02.1998**
(21) Anmeldenummer: 97113789.8
(22) Anmeldetag: 08.08.1997
(51) Int. Cl.: C07C 209/82, C07C 211/03

(54) **Verfahren zur Herstellung von primären Aminen**

(30) Priorität: 08.08.1996 DE 19632107
(71) Anmelder: BASF AKTIENGESELLSCHAFT, 67056 Ludwigshafen (DE)
(72) Erfinder: Mundinger, Klaus, Dr., 67117 Limburgerhof (DE); Schneider, Rolf, Dr., 68163 Mannheim (DE)
(74) Vertreter: Kinzebach, Werner, Dr.

(57) **Zusammenfassung**

Die Erfindung betrifft ein Verfahren zur Herstellung von Aminen mit an sekundäre oder tertiäre Kohlenstoffatome gebundenen Aminogruppen durch Umsetzung eines Carbenium-Ionen-Precursors mit einem Nitril in Gegenwart einer starken Säure, wobei man das Amin aus dem Salz mit der starken Säure mit Ammoniak oder Aminen neutralisiert.

## Beschreibung

Die vorliegende Erfindung betrifft ein Verfahren zur Herstellung von primären Aminen mit an sekundäre oder tertiäre C-Atome gebundenen Aminogruppen.

Die Anlagerung von Aminogruppen an sekundäre bzw. tertiäre C-Atome erfolgt im allgemeinen über die sogenannte Ritter-Reaktion, die z.B. aus Organic Reactions, 1969, Band 17, S. 215-325 bekannt ist.

Die Ritterreaktion umfaßt die nukleophile Addition eines Nitrils an ein Carbeniumion in Gegenwart einer starken Säure und nachfolgende Hydrolyse des intermediär gebildeten Formamids zum Anin, wie in den Reaktionsgleichungen A, B und C gezeigt.

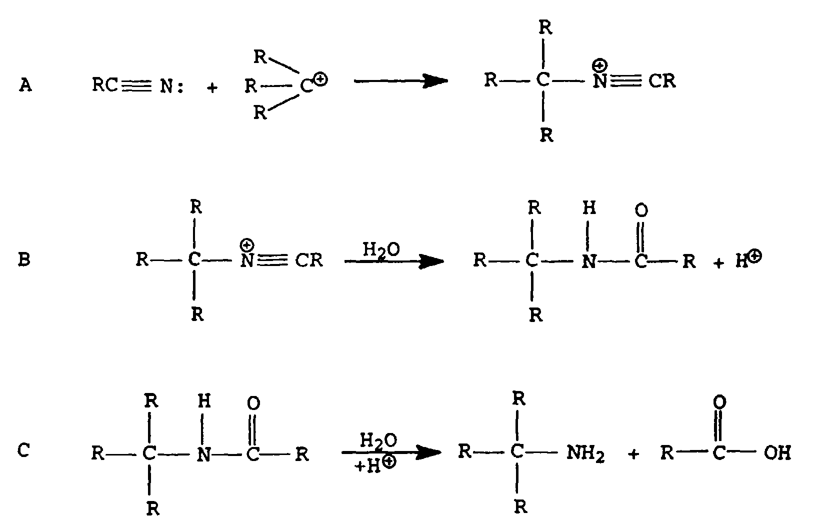

Als Edukte eignen sich folglich solche Verbindungen, die Carbeniumionen bilden können.

Zur Isolierung der Amine aus der sauren Lösung wird in der Regel mit Alkali, z.B. NaOH neutralisiert. Dabei fällt jedoch eine große Menge nicht verwertbarer Abfallsalze an, die deponiert werden müssen und eine großtechnische Produktion somit verteuern.

Des weiteren können über das zu entsorgende Abwasser organische Verbindungen in die Natur gelangen, die biologisch sehr schlecht abbaubar sind.

Der Erfindung liegt daher die Aufgabe zugrunde, ein Verfahren zur Herstellung der obengenannten Verbindungsklasse zu ermöglichen, das ohne die Bildung nicht verwertbarer Abfallsalze verläuft. Außerdem soll das Verfahren keine biologisch schlecht abbaubaren Abwässer ergeben.

Überraschenderweise wurde gefunden, daß diese Aufgabe gelöst wird, wenn man die schwefelsaure Aminlösung mit Ammoniak neutralisiert. Bekanntlich sind alle Amine (primäre, sekundäre und tertiäre) basischer als Ammoniak (vgl. Beyer-Walter, 21. Aufl., 1988, S. 158 ff.). Es ist also nicht zu erwarten, daß Ammoniak die basischeren Amine aus ihren Salzen verdrängen kann. Es ist gleichfalls überraschend, daß die schwefelsaure Aminlösung durch tertiäre Alkylamine, wie z.B. Trimethylamin, die deutlich weniger basisch sind, als die primären Amine der Formel I, neutralisiert werden kann.

Die hierbei entstehenden ammoniumsalzhaltigen Lösungen sind einer thermischen Spaltung zugänglich, so daß keine Abfallsalze oder schlecht abbaubare Abwässer zurückbleiben.

Gegenstand der Erfindung ist somit ein Verfahren zur Herstellung von Aminen der Formel I, 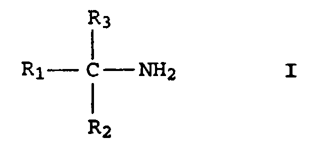 in der R₁, R₂ und R₃ unabhängig voneinander für H oder eine Alkyl-, Cycloalkyl-, Alkylcycloalkyl-, Cycloalkylalkyl-, Hydroxyalkyl-, Hydroxycycloalkyl-, Arylalkyl- oder Arylgruppe stehen, wobei nur einer der Reste R₁, R₂ und R₃ für ein Wasserstoffatom stehen kann, oder R₁ für Wasserstoff steht und R₂ und R₃ zusammen mit dem Kohlenstoffatom, an das sie gebunden sind, einen Cycloalkylring bilden, durch Umsetzung eines Carbenium-Ionen-Precursors, der in der Lage ist ein Carbeniumion der Formel R₁R₂R₃C^{⊕} zu bilden, mit einem Nitril in Gegenwart einer starken Säure, das dadurch gekennzeichnet ist, daß man das Amin aus dem Salz mit der starken Säure mit Ammoniak oder einem Amin freisetzt.

Hierbei steht Alkyl vorzugsweise für unverzweigtes oder verzweigtes C₁- bis C₁₂-Alkyl, insbesondere C₁-C₆-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, tert.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl;
Cycloalkyl steht vorzugsweise für C₃- bis C₈-Cycloalkyl, nämlich Cyclopropyl, Cyclobutyl, Cyclopentyl, Cyclohexyl, Cycloheptyl und Cyclooctyl;
Alkylcycloalkyl steht vorzugsweise für Cycloalkyl wie oben definiert, das durch mindestens eine C₁-C₆-Alkylgruppe substituiert ist, z.B. Methylcyclohexyl und Isopropylcyclohexyl;
Cycloalkylalkyl steht vorzugweise für Cyclopropylmethyl, Cyclopentylmethyl, Cyclohexylmethyl, Cyclopropylethyl, Cyclopentylethyl und Cyclohexylethyl;
Hydroxyalkyl steht vorzugsweise für C₁- bis C₈-Hydroxyalkyl, wie Hydroxymethyl, Hydroxyethyl, 1-Hydroxypropyl, 2-Hydroxypropyl, 1-Hydroxybutyl, 2-Hydroxybutyl, 1-Hydroxypentyl, 2-Hydroxypentyl, 1-Hydroxyhexyl, 2-Hydroxyhexyl, 1-Hydroxyheptyl, 2-Hydroxyheptyl, 1-Hydroxyoctyl und 2-Hydroxyoctyl;
Hydroxycycloalkyl steht vorzugsweise für C₅- bis C₈-Hydroxycycloalkyl, wie Hydroxycyclopentyl und Hydroxycyclohexyl;
Arylalkyl steht vorzugsweise für C₇-C₂₀-Arylalkyl, wie Benzyl, Phenethyl und Phenyldecyl.

Aryl steht vorzugsweise für Phenyl, 1-Naphthyl oder 2-Naphthyl. Aryl kann gegebenenfalls durch 1 bis 3 Alkyl-, Alkoxy- oder Hydroxygruppen substituiert sein.

Der Carbenium-Ionen-Precursor ist vorzugsweise ausgewählt unter Verbindungen der Formel II 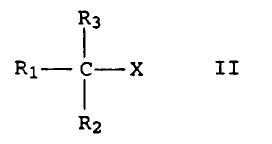 worin R₁ bis R₃ die oben angegebenen Bedeutungen besitzen und X für H, OH, Halogen, COOR oder OCOR steht, wobei R für Alkyl oder Aryl steht, oder zusammen mit X für einen Alkylidenrest steht und R₁ und R₂ die oben angegebenen Bedeutungen besitzen. Alkyliden steht vorzugsweise für C₁-C₈-Alkyliden, wie Methyliden, Ethyliden oder Propyliden. Besonders bevorzugte Carbenium-Ionen-Precursoren sind jedoch Alkene der Formel IIa, 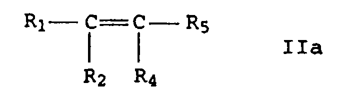 in der R₁ und R₂ die oben angegebenen Bedeutungen besitzen und R₄ und R₅ unabhängig voneinander für H oder eine Alkyl-, Cycloalkyl-, Hydroxyalkyl-, Arylalkyl- oder Arylgruppe stehen, wobei nur einer der Reste R₁ und R₂ für Wasserstoff stehen kann; und Alkohole der Formel IIb, 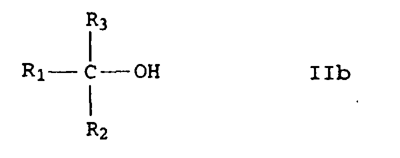 in der R₁, R₂ bzw. R₃ die oben angegebenen Bedeutungen besitzen.

Das Amin der Formel I liegt in dem erhaltenen Reaktionsgemisch als Salz mit der verwendeten starken Säure vor. Die Freisetzung des Amins aus dem Salz erfolgt durch Zugabe von Ammoniak oder einem Anin. Ammoniak wird zweckmäßigerweise als wäßrige Lösung, insbesondere als höher konzentrierte Lösung, z.B. 20-30%ig, zugegeben werden. Man kann Ammoniak aber auch als Gas einleiten oder in flüssiger Form zugeben.

Brauchbare Amine sind insbesondere sekundäre und tertiäre Alkylamine oder Hydroxyalkylamine, wie Trimethylamin, Triethylamin, Dimethylamin, Diethylamin, Diethanolamin oder Triethanolamin. Außerdem sind aminhaltige Aufarbeitungsrückstände aus technischen Prozessen, deren pK_{b}-Werte im Bereich von etwa 1 bis 5 liegen, brauchbar.

Die starke Säure ist vorzugsweise ausgewählt unter anorganischen Säuren, insbesondere Perchlorsäure, Phosphorsäure, Polyphosphorsäure und Schwefelsäure, wobei Schwefelsäure besonders bevorzugt ist. Man kann auch starke organische Säuren einsetzen, beispielsweise Sulfonsäuren.

Als Nitril ist Cyanwasserstoff besonders bevorzugt.

Die Reaktion wird in üblicher Weise durchgeführt. Sie kann in einem üblichen kühl- und beheizbaren Reaktionsgefäß, beispielsweise einem Rührreaktor, der gegebenenfalls mit einer herkömmlichen Destillationsapparatur (z.B. Kolonne mit Siebböden, Raschigringen, gerichteten Packungen etc.) versehen ist, kontinuierlich oder diskontinuierlich erfolgen, wobei die kontinuierliche Reaktionsführung bevorzugt ist. In der Regel wird bei Normaldruck gearbeitet, man kann jedoch auch unter Druck arbeiten. Im allgemeinen geht man so vor, daß zunächst die starke Säure, Wasser und das Nitril, gegebenenfalls unter Kühlung, vermischt werden, wobei das Nitril im allgemeinen im Überschuß (10-20 Mol-%) verwendet wird. Zu dieser Mischung wird dann der Carbenium-Ionen-Precursor gegeben.

Das molare Verhältnis zwischen starker Säure und dem Carbenium-Ionen-Precursor beträgt ≥ 1:1, vorzugsweise 1,2 bis 1,6:1. Reaktionstemperatur und die Reaktionszeit können in einem weiten Bereich variieren. Im allgemeinen beträgt die Reaktionszeit 1 bis 10 Stunden und die Reaktionstemperatur liegt im Bereich von etwa -20°C bis 80°C. Zur Verseifung des gebildeten Amids und des überschüssigen Nitrils wird dann Wasser zugegeben. Anschließend werden gegebenenfalls leichtsiedende Bestandteile abdestilliert. Zu der verbliebenen Lösung wird dann Ammoniak oder ein Amin gegeben, um das Amin der Formel I freizusetzen. Man gibt mindestens zwei Moläquivalente Ammoniak bzw. Amin bezüglich der eingesetzten starken Säure zu. Zweckmäßigerweise arbeitet man bei einer Temperatur im Bereich von etwa 0°C bis 60°C, vorzugsweise 20°C bis 50°C. Im Anschluß an die Zugabe von Ammoniak bzw. des Amins wird das Wertprodukt als organische Phase von der ammoniumsalzhaltigen Lösung abgetrennt. Im Regelfall erfolgt die Phasentrennung bei Raumtemperatur, bei der Herstellung längerkettiger Alkylamine (mit 8 oder mehr C-Atomen) erfolgt die Phasentrennung vorteilhaft bei Temperaturen ≥ 50°C. Gegebenenfalls wird das Wertprodukt mit geeigneten organischen Lösungsmitteln extrahiert. Für die Extraktion geeignete Lösungsmittel sind beispielsweise chlorierte Kohlenwasserstoffe wie Dichlormethan oder Chlorbenzol, Ether wie z.B. Diethylether oder tert.-Butylmethylether, Ester wie z.B. Essigsäureethylester, Ketone wie z.B. Aceton, Diethylketon oder Methylethylketon oder Alkohole wie z.B. Pentanol oder Ethylhexanol bzw. Gemische derartiger Lösungsmittel. Die Extraktion kann in an sich bekannter Weise durchgeführt werden. Vorzugsweise extrahiert man im Gegenstrom, z.B. in Kolonnen ohne Energieeintrag, gepulsten Kolonnen, Kolonnen mit rotierenden Einsätzen und insbesondere Mixer-Settler-Apparaturen. Die anfallende organische Phase wird gegebenenfalls in üblicher Weise, wie z.B. durch Destillation weiter aufgearbeitet.

Die ammoniumsalzhaltigen Lösungen, die gegebenenfalls noch Spuren an Amin der Formel I enthalten, können einer thermischen Spaltung unter Bildung von Stickstoff, Wasser, gegebenenfalls Kohlendioxid und Schwefeldioxid zugeführt werden. Diese Spaltung erfolgt im allgemeinen bei Temperaturen > 1000°C, sie ist beschrieben in Ullmann, Band 21, 4. Auflage, S. 48 ff. Aus dem Schwefeldioxid kann in bekannter Weise Schwefelsäure gebildet werden, die erneut in den Prozeß eingesetzt werden kann. Es bleiben keine Abfallsalze oder schlecht abbaubare Abwässer zurück.

Die folgenden Beispiele erläutern die Erfindung, ohne sie jedoch darauf einzuschränken:

### Beispiel 1: Synthese eines C₁₂-Amin-Gemisches

In einen 2-l-Dreihalskolben werden 280 g (2,8 mol) H₂SO₄ (konz.) bei Raumtemperatur vorgelegt, anschließend gibt man nacheinander 43,2 g (2,4 mol) Wasser und 65 g (2,4 mol) HCN unter Kühlung (0 bis 10°C) innerhalb von 40 min zu.

336 g (2 mol) Tetramerpropylen (Isomerenmischung) werden innerhalb 60 min bei 15 bis 20°C zudosiert. Die Reaktionswärme wird durch CO₂/Methanolkühlung abgeführt.

Die viskose Reaktionsmischung wird 2 h bei 30°C und anschließend 3 h bei 50°C nachgerührt.
Zur Verseifung des Formamids und der überschüssigen HCN werden 840 ml Wasser in die Reaktionslösung dosiert, wodurch eine Temperaturerhöhung auf 40°C erfolgt.

Man heizt auf 100°C auf und destilliert dabei gleichzeitig 29,6 g organischen Leichtsieder ab. Nach 4 h bei ca. 100°C wird die homogene Lösung auf 50°C abgekühlt und mit 544 g (8 mol) NH₃-Lösung (25%ig) auf pH 9 gestellt. Nach Beendigung der NH₃-Zugabe wird die zweiphasige Mischung im Phasenscheider getrennt und die organische Phase bei 50 mbar destilliert. Das C₁₂-Amin-Gemisch siedet bei 108 bis 125°C.

Man erhält 328 g (1,77 mol) eines C₁₂-Amin-Gemisches, dies entspricht einer Ausbeute von 88,5%, bezogen auf eingesetztes Tetramerpropylen.

### Beispiel 2:

In einen 2-l-Dreihalskolben werden nacheinander 54 g (3 mol) Wasser, 300 g (3,0 mol) H₂SO₄ (konz.) bei Raumtemperatur vorgelegt und anschließend 65 g (2,4 mol) HCN unter Kühlung (0 bis 10°C) innerhalb von 40 min zudosiert.

224 g (2 mol) 2,4,4-Trimethylpenten (Isomerenmischung) werden innerhalb 60 min bei 15 bis 20°C zudosiert. Die Reaktionswärme wird durch CO₂/Methanolkühlung abgeführt.

Die viskose Reaktionsmischung wird 2 h bei 30°C und anschließend 3 h bei 50°C nachgerührt.

Zur Verseifung des Formamids und der überschüssigen HCN werden 800 ml Wasser in die Reaktionslösung dosiert, wodurch eine Temperaturerhöhung auf 40°C erfolgt.

Man heizt auf 100°C auf und destilliert dabei gleichzeitig 16,9 g organische Leichtsieder ab.

Nach 4 h bei ca. 100°C wird die homogene Lösung auf 50°C abgekühlt und mit 155 g (9,1 mol) NH₃ auf pH 9 gestellt. Nach Beendigung der NH₃-Zugabe wird die zweiphasige Mischung bei 50°C mit 250 ml Cyclohexan extrahiert und die Phasen im Phasenscheider getrennt. Die organische Phase wird destillativ aufgearbeitet. Das 1,1,3,3-Tetramethylbutylamin siedet bei 200 mbar bei 74°C.

Man erhält 219 g (1,7 mol) 1,1,3,3-Tetramethylbutylamin, dies entspricht einer Ausbeute von 84,8%, bezogen auf eingesetztes 2,4,4-Trimethylpenten.

## Patentansprüche

1. Verfahren zur Herstellung von Aminen der Formel I, 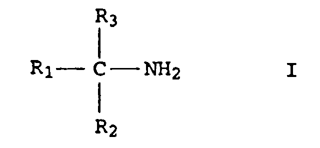 in der R₁, R₂ und R₃ unabhängig voneinander für H oder eine Alkyl-, Cycloalkyl-, Alkylcycloalkyl-, Cycloalkylalkyl-, Hydroxyalkyl-, Hydroxycycloalkyl-, Arylalkyl- oder Arylgruppe stehen, wobei nur einer der Reste R₁, R₂ und R₃ für ein Wasserstoffatom stehen kann, oder R₁ für Wasserstoff steht und R₂ und R₃ zusammen mit dem Kohlenstoffatom, an das sie gebunden sind, einen Cycloalkylring bilden, durch Umsetzung eines Carbenium-Ionen-Precursors, der in der Lage ist ein Carbeniumion der Formel R₁R₂R₃C^{⊕} zu bilden, mit einem Nitril in Gegenwart einer starken Säure, dadurch gekennzeichnet, daß man das Amin aus dem Salz mit der starken Säure mit Ammoniak oder einem Amin freisetzt.

2. Verfahren nach Anspruch 1, dadurch gekennzeichnet, daß der Carbenium-Ionen-Precursor ausgewählt ist unter Verbindungen der Formel II 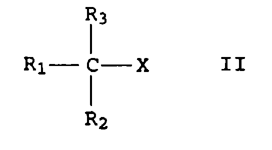 worin R₁ bis R₃ die oben angegebenen Bedeutungen besitzen und X für H, OH, Halogen, COOR oder OCOR steht, wobei R für Alkyl oder Aryl steht, oder zusammen mit X für einen Alkylidenrest steht und R₁ und R₂ die oben angegebenen Bedeutungen besitzen.

3. Verfahren nach Anspruch 1 oder 2, dadurch gekennzeichnet, daß die starke Säure ausgewählt ist unter Perchlorsäure, Phosphorsäure, Polyphosphorsäure, Sulfonsäuren und Schwefelsäure.

4. Verfahren nach einem der vorhergehenden Ansprüche, dadurch gekennzeichnet, daß der Carbenium-Ionen-Precursor ein Alken der Formel IIa, 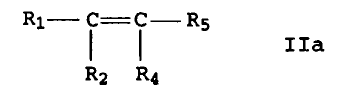 in der R₁ und R₂ die in Anspruch 1 angegebenen Bedeutungen besitzen und R₄ und R₅ unabhängig voneinander für H oder eine Alkyl-, Cycloalkyl-, Hydroxyalkyl-, Arylalkyl- oder Arylgruppe stehen, wobei nur einer der Reste R₁ und R₂ für Wasserstoff stehen kann; oder ein Alkohol der Formel IIb ist, 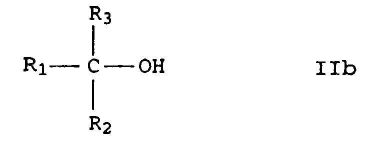 in der R₁, R₂ bzw. R₃ die in Anspruch 1 angegebenen Bedeutungen besitzen.

5. Verfahren nach einem der vorhergehenden Ansprüche, dadurch gekennzeichnet, daß man das Reaktionsgemisch mit Ammoniak neutralisiert.

6. Verfahren nach Anspruch 7, dadurch gekennzeichnet, daß man die ammoniumsalzhaltige neutralisierte Lösung nach Abtrennung des Amins der Formel I einer thermischen Spaltung bei Temperaturen von > 1000°C zuführt.

7. Verfahren nach einem der vorhergehenden Ansprüche, dadurch gekennzeichnet, daß man das Nitril in überstöchiometrischen Mengen zusetzt.

8. Verfahren nach einem der vorhergehenden Ansprüche, dadurch gekennzeichnet, daß man als Nitril Cyanwasserstoff verwendet.
